# FASCICULE DE BREVET EUROPEEN

(11) **EP 1 206 247 B1**
(45) Date de publication et mention de la délivrance du brevet: **02.12.2009**
(21) Numéro de dépôt: 00956599.5
(22) Date de dépôt: 02.08.2000
(51) Int. Cl.: A61K 9/20, A61K 9/00, A61K 9/50

(54) **COMPOSITION PHARMACEUTIQUE FLOTTANTE COMPRENANT UNE PHASE ACTIVE ET UNE PHASE NON ACTIVE**
SCHWIMMENDE PHARMAZEUTISCHE ZUSAMMENSETZUNG MIT EINER AKTIVEN PHASE UND EINER INAKTIVEN PHASE
FLOATING PHARMACEUTICAL COMPOSITION COMPRISING AN ACTIVE PHASE AND A NON-ACTIVE PHASE

(30) Priorité: 06.08.1999 FR 9910285
(43) Date de publication de la demande: 22.05.2002
(73) Titulaire: GALENIX DEVELOPPEMENT, 33127 Saint-Jean d'Illac (FR)
(72) Inventeur: BESSE, Jérôme, F-33480 Listrac Medoc (FR)
(74) Mandataire: Augarde, Eric
(86) Numéro de dépôt international: PCT/FR2000/002223
(87) Numéro de publication internationale: WO 2001/010417

(56) Documents cités:
- EP-A- 0 235 718
- EP-A- 0 667 151
- EP-A- 0 669 129
- EP-A- 0 795 324
- FR-A- 2 766 708
- US-A- 5 783 212

## Description

La présente invention est relative à une composition pharmaceutique flottante constituée d'au moins une première phase comprenant au moins un principe actif très fortement dosé en association avec un ou plusieurs excipients et d'au moins une seconde phase comprenant au moins un système générateur d'un gaz.

L'invention concerne aussi des comprimés comprenant une telle composition pharmaceutique ainsi qu'un procédé de préparation de tels comprimés.

Diverses compositions pharmaceutiques formulées sous la forme de comprimés flottants sont connues dans l'état de la technique.

Par exemple, des comprimés flottants ont été obtenus en agissant sur la densité de la composition totale, par exemple en faisant varier les rapports pondéraux entre différents polymères excipients tels que la carboxyméthylcellulose, l'éthylcellulose, la povidone réticulée, ou encore le chitosan, composé bien connu pour sa faible densité et pour ses propriétés muco-adhésives.

Une autre solution technique a consisté à utiliser, parmi les excipients, une matière minérale poreuse telle que le silicate de calcium, dans laquelle de l'air a été emprisonné dans les pores, les comprimés étant alors recouverts d'un polymère tel que l'hydroxypropylcellulose ou encore l'éthylcellulose destiné à retenir l'air au sein de la matrice minérale poreuse.

On a également eu recours dans l'état de la technique à des capsules de gélatine remplies d'un gaz inerte ou encore remplies d'une substance contenant des bulles de gaz inerte.

Une autre solution technique a consisté à mettre en oeuvre des microsphères de résine de polycarbonate creux chargé avec le principe actif ou encore des microsphères creuses contenant le principe actif en présence d'Eudragit-S, qui sont des sphères parfaites de 500 à 1000 µm de diamètre capables de flotter plus de douze heures dans un milieu acide dans une solution de Tween-20 à 0.02%.

Une autre stratégie a consisté à obtenir une diminution de la densité du comprimé au moment de l'emploi, par l'incorporation dans la composition pharmaceutique de composés capables de libérer un gaz qui reste, au moins en partie, emprisonné au sein de ce comprimé et qui va ainsi lui permettre de flotter à la surface du suc gastrique, dans l'estomac. Les demandes EP 0 795 324 et EP 0 235 718 divulguent des compositions pharmaceutiques à résidence gastrique multicouches comprenant un principe actif et des polymères hydrophiles.

La demande PCT n°WO 98/47.506 décrit une composition pharmaceutique à résidence gastrique comprenant un principe actif de la famille des benzamides en association avec un polymère hydrophile ou une matrice minérale poreuse et un système générateur de dioxyde de carbone. Une telle composition pharmaceutique peut convenir pour de faibles doses de principe actif, en particulier lorsque la dose de principe actif dans le comprimé ne dépasse pas 500 mg, comme c'est le cas en général pour les benzamides. En revanche, il a été observé pour de telles compositions une modification du profil de dissolution du principe actif, par rapport à des compositions pharmaceutiques conventionnelles ne contenant pas de système générateur de gaz.

De plus, le fait que le système générateur de gaz et le principe actif soient sous la forme d'un mélange intime est de nature à favoriser les interactions entre le principe actif et les autres ingrédients de la composition pharmaceutique, ce qui peut entraîner une altération chimique, au moins partielle, du principe actif et en conséquence une diminution significative de l'efficacité thérapeutique de la composition ainsi que des problèmes de conservation à long terme.

ICHIKAWA et al. (1991, Journal of Pharmaceutical Sciences, vol.80, n°11, pages 1062-1066) ont décrit un comprimé flottant constitué d'un noyau contenant une composition pharmaceutique à libération prolongée recouvert d'une première couche comprenant un couple effervescent, cette première couche étant elle-même recouverte d'une pellicule de polymère capable de retenir un gaz. L'eau ou le suc gastrique pénètre à travers la pellicule de polymère externe et entre en contact avec le couple effervescent qui génère alors le dioxyde de carbone . En se libérant au sein du comprimé, le dioxyde de carbone va gonfler le comprimé sous la forme d'un « micro-ballon » capable de flotter dans l'estomac. Un tel comprimé a le désavantage d'être difficile à industrialiser et est en outre onéreux à fabriquer. De plus, le pelliculage de la couche de couple effervescent par le polymère nécessite l'utilisation de solvants autres que l'eau, tels que l'éthanol, le dichlorométhane ou encore l'acétone, afin d'éviter que le couple effervescent ne réagisse au moment de la fabrication.

Outre le coût, l'emploi de solvants organiques pose le problème de leur élimination dans les étapes finales de fabrication du comprimé, de manière à satisfaire aux contraintes administratives croissantes préalables à l'obtention d'une autorisation de mise sur le marché d'une spécialité pharmaceutique.

La présente invention remédie aux nombreux inconvénients identifiés ci-avant des compositions flottantes de l'état de la technique et fournit une nouvelle composition pharmaceutique comprenant au moins une phase active et une phase non active.

Ainsi, un premier objet de l'invention consiste en une composition pharmaceutique flottante telle que defini dans les revendications.

Selon un premier aspect, la phase active et la phase non active ont une surface commune de contact.

Selon un second aspect, la phase active et la phase non active sont séparées par une couche de polymère.

Dans tous les cas, la phase active et la phase inactive sont comprises dans une structure unique, par exemple celle d'un comprimé.

Dans un mode de réalisation préféré la phase non active est en contact direct avec le milieu extérieur, par exemple le suc gastrique.

Plus précisément, dans le cas où la composition pharmaceutique est formulée sous la forme de comprimés, la phase active et la phase non active sont solidarisées entre elles par compression, selon des techniques bien connues de l'homme du métier.

La nature de la phase non active de la composition pharmaceutique selon l'invention doit être telle qu'elle permet la rétention des bulles de gaz produites par le système générateur de gaz inclus dans cette dernière, pendant un temps suffisant pour permettre la flottaison de ladite composition pharmaceutique à l'interface liquide/air, lors du passage dans le tractus gastro-intestinal.

C'est la raison pour laquelle la seconde phase non active est formée d'un micro-réseau de polymère(s) hydrophile(s).

Le système générateur d'un gaz est rendu nécessaire pour diminuer la forte densité de la composition pharmaceutique de l'invention, cette forte densité résultant de forte proportion en principe(s) actif(s) dans la phase active de la composition.

De plus, le système générateur d'un gaz permet la libération rapide du gaz, dès la mise en contact de ce système avec le milieu aqueux gastrique ou post-gastrique, provoquant rapidement une diminution de densité de la composition pharmaceutique avec une vitesse de réaction bien supérieure à celle qui serait observée avec des polymères gonflants. L'utilisation de polymères gonflants ne permet donc pas de résoudre le problème technique de l'invention, car leur vitesse de gonflement n'est pas suffisante et ne permettrait pas de réduire suffisamment la densité d'une composition pharmaceutique fortement dosée en principe(s) actif(s) de l'invention.

En outre, dans un mode de réalisation préféré, au moins l'une des surfaces de la phase non active est directement en contact avec le milieu extérieur. Cela signifie que la phase non active n'est revêtue d'aucun pelliculage empêchant tout contact avec le milieu extérieur.

Selon un premier mode de réalisation, le polymère hydrophile de la phase non active est choisi parmi des substances polysaccharidiques, les substances apparentées, et les substances protéiques.

Avantageusement, les substances saccharidiques et les substances apparentées sont choisies parmi les galactomannanes et ses dérivés, l'amidon et ses dérivés, la gomme arabique, la gomme adragante, les pectines et ses dérivés, les alginates, la cellulose et ses dérivés, les dextrans de haut poids moléculaire, ou encore le xanthane et ses dérivés.

Parmi ces différentes substances, plusieurs produits commerciaux peuvent être mis en oeuvre comme par exemple METOLOSE® 90 SH 4000 SR (hypromellose ou KLUCEL^{®} HXF (hydroxypropylcellulose).

De manière préférée, les substances protéiques sont choisies parmi la gélatine et ses produits dérivés purifiés.

Selon un autre aspect, le polymère de la phase non active est choisi parmi les poloxamères, les polyéthylèneglycol de haut poids moléculaire (supérieur à 6000 kDa) ou encore les polymères des acides acryliques ou méthacryliques et leurs dérivés.

De manière préférée, le polymère ou le mélange de polymères constitutif de la phase non active forme un « gel » au contact d'un milieu aqueux. Il y a une hydratation progressive des particules de polymère avec un léger gonflement de celles-ci, qui se dissolvent ensuite pour former un « gel ». Le gel gonfle du fait du déplacement de l'eau du milieu aqueux dans la phase non active du comprimé.

La phase non active de la composition pharmaceutique selon l'invention comprend en outre un moyen destiné à diminuer la densité de cette dernière au moment de son administration chez l'homme ou l'animal, ce moyen étant constitué d'un système générateur d'un gaz, tel qu'un système générateur de dioxyde de carbone.

Un système générateur de dioxyde de carbone préférentiellement utilisé au sein de la phase non active d'une composition pharmaceutique selon l'invention comprend un carbonate de métal alcalin ou alcalino-terreux, ou encore un bicarbonate de métal alcalin.

Comme carbonate de métal alcalin, on préférera le carbonate de calcium.

Avantageusement, le bicarbonate de métal alcalin est le bicarbonate de sodium ou le bicarbonate de potassium.

Un système générateur de dioxyde de carbone, d'un type tel que défini ci-dessus, ne produit de dioxyde de carbone que lorsqu'il se retrouve en contact avec un milieu aqueux acide, tel que le suc gastrique présent dans la cavité stomacale. Il s'agit d'un système générateur de dioxyde de carbone dit « pH dépendant ».

Selon un autre mode de réalisation, la phase non active de la composition pharmaceutique selon l'invention peut contenir un système générateur de gaz, en particulier de dioxyde de carbone, dit « pH indépendant ».

Dans ce mode de réalisation particulier, un système générateur de dioxyde de carbone comprend, en mélange intime, un carbonate de métal alcalin ou alcalino-terreux ou encore un bicarbonate de métal alcalin, tel que défini ci-dessus, en association avec un acide choisi parmi les acides mono- et poly-carboxyliques ou encore un sel partiel d'un acide polycarboxylique. Un tel mélange est communément appelé un couple effervescent.

Par sel partiel d'un acide carboxylique, on entend selon l'invention, un composé polycarboxylique dans lequel seule une partie des fonctions carboxyliques du composé acide est associée à des cations.

A titre d'exemple d'acides carboxyliques, on peut citer l'acide citrique, l'acide tartrique et leurs sels partiels tel le citrate monosodique et/ou disodique.

Dans le cas d'une utilisation d'un système générateur de dioxyde de carbone pH indépendant, on aura avantageusement recours à un mélange dans lequel le rapport entre le carbonate de métal alcalin ou alcalino-terreux ou le bicarbonate de métal alcalin, d'une part, et l'acide mono- ou poly-carboxylique, sous forme saline ou non saline, d'autre part, est approximativement stoechiométrique, selon les usages classiques bien connus de l'homme du métier.

Selon un autre aspect, la phase non active comprend de 10 à 70% en poids de ladite phase du polymère hydrophile.

Selon un mode de réalisation préféré, la phase non active comprend de 5 à 60% en poids de ladite phase du système générateur de gaz, que celui-ci soit pH dépendant ou pH indépendant.

Comme déjà mentionné précédemment, la phase active de la composition pharmaceutique flottante selon l'invention comprend au moins un principe actif en association avec un ou plusieurs excipients.

La constitution de cette phase active peut être de toute nature connue de l'homme du métier. En effet, il a été montré que le profil de dissolution de la phase active au sein d'une composition pharmaceutique flottante selon l'invention, c'est-à-dire en association avec la phase non active, n'était pas significativement modifié par rapport au profil de dilution observé de la phase active seule.

En particulier, la phase active peut contenir un seul principe actif ou au contraire plusieurs principes actifs en association, le ou les principes actifs pouvant être de toute nature.

Toutefois, les propriétés particulières de flottaison de la composition pharmaceutique selon l'invention la rend particulièrement apte à l'utilisation de principes actifs ayant une biodisponibilité faible ou irrégulière, due par exemple à une dissolution lente.

En effet, de tels principes actifs, lorsqu'ils sont inclus dans des compositions conventionnelles, non flottantes, sont libérés pendant un temps restreint dans la cavité de l'estomac, puis transitent rapidement dans le tractus intestinal où la libération dudit principe actif se poursuit.

Ainsi, dans le cas de principes actifs dont les sites cibles sont localisés dans l'estomac ou dans la partie haute de l'intestin grêle, des compositions pharmaceutiques conventionnelles ne permettent pas une biodisponibilité du principe actif suffisante pour saturer ces sites, de nature à permettre une action thérapeutique optimale.

Il est en effet généralement observé qu'un comprimé classique ne réside dans la cavité stomacale que pendant un temps variant de 1 à 3 minutes, au-delà duquel ce comprimé franchit le pylhore et passe alors au niveau de l'intestin grêle et transite tout au long du tractus intestinal jusqu'au colon.

Au contraire, un comprimé selon l'invention flotte au niveau du suc gastrique dans l'estomac.

Il a été montré in vitro que les comprimés constitués d'une composition pharmaceutique selon l'invention étaient capables de flotter après environ 30 secondes de contact suivant le moment de leur immersion dans un milieu aqueux acide et étaient en outre capables de flotter pendant environ 10 à 20 heures.

Il en résulte que le principe actif contenu dans la phase active est biologiquement accessible aux sites cibles pendant un temps prolongé, d'une part, et que la totalité de la dose de principe actif contenue initialement dans la phase active peut accéder à ces sites cibles, d'autre part.

Avantageusement, une composition pharmaceutique selon l'invention comprend un ou plusieurs principes actifs à fenêtre d'absorption à action locale.

En effet, un comprimé d'une composition pharmaceutique selon l'invention, du fait de sa flottaison, permet la libération du ou des principes actifs contenus dans la phase active en amont ou sur des sites cibles de ces derniers, pendant une longue période de temps, et la saturation des sites cibles pendant un temps bien supérieur aux comprimés non flottants de l'état de la technique. Il est particulièrement tiré profit de ces caractéristiques de flottaison d'un comprimé de l'invention lorsqu'une libération bimodale est recherchée. La localisation du comprimé en amont des sites cibles rend particulièrement avantageux une libération immédiate d'une forte concentration du ou des principes actifs contenus dans la phase active afin de saturer rapidement les sites cibles, suivie d'une libération prolongée d'une quantité plus faible du ou des principes actifs afin de maintenir pendant une longue période de temps l'état de saturation des sites cibles.

En outre, une libération de la totalité de la quantité du ou des principes actifs en amont des sites cibles exclut pratiquement, ou à tout le moins diminue fortement, une déviation des voies métaboliques qui est observée avec les comprimés non flottants. Avec ces derniers, une quantité significative du ou des principes actifs est libérée en aval des sites cibles, ledit ou lesdits principes actifs étant alors transformés par des voies métaboliques différentes des voies métaboliques ciblées initialement.

Les principes actifs pouvant être inclus dans une composition pharmaceutique selon l'invention sont par exemple le Flutamide ou la Metformine.

En outre, on utilisera avantageusement un ou plusieurs principes actifs capables de s'ioniser principalement dans le tractus gastro-intestinal ainsi que des principes actifs qui, parce qu'ils sont libérés très lentement, ne peuvent saturer les sites enzymatiques cibles lorsqu'ils sont formulés avec des compositions pharmaceutiques conventionnelles.

Selon un autre aspect, on utilisera un ou plusieurs principes actifs ayant une action locale au niveau des muqueuses digestives. Appartiennent à cette classe de principes actifs les anti-acides, comme par exemple l'hydroxyde de magnésium, qui sont utilisés sous une forme hautement dosée.

Plus généralement, une composition pharmaceutique selon l'invention comprend un ou plusieurs principes actifs choisis parmi les principes actifs suivants:
- antibiotiques: céfalosporines (cefaclor)
- antisécrétoires gastriques: métoclopramide
- anticonvulsivants: acide valproïque et ses dérivés, carbanazepine,
- antiviraux: nucléosides (acyclovir),
- inhibiteurs calciques: nicardipine et ses dérivés, virapamil
- diurétiques: sulfamides (furosémide)
- antiarythmiques: dihydroquinidine
- suppléments minéraux : potassium
- antidiabétiques: biguanidine (metformine et ses dérivés),
- anti-inflammatoires: etodolac, kétoproféne, ibuprofène,
- antihormones: antiandrogènes (flutamide)
- antiparkinsoniens: levodopa/carbidopa
- bétabloquants: acébutolol
- neuroleptiques: butyrophénones (penfluridol), phénothiazines,

Selon l'invention, la phase active est fortement dosée en principe(s) actif(s).

Préférentiellement, la phase active comprend une quantité de principe actif d'au moins environ 80%, préférentiellement d'au moins 85% et encore plus préférentiellement d'au moins environ 90% en poids de la phase active.

Selon ce mode de réalisation particulier, la composition pharmaceutique selon l'invention est en outre **caractérisée en ce que** la phase active est constituée d'une composition comprenant au moins un principe actif et, à titre d'agents désintégrants, de l'hydroxypropylméthylcellulose, de l'hydroxypropylcellulose fortement substitué et/ou de l'éthylcellulose dans des quantités inférieures à 15% en poids de la phase active et sous une forme permettant d'obtenir un effet désintégrant tout en évitant la formation d'un réseau continu.

La constitution d'une phase active telle que définie ci-dessus est décrite en détail dans la demande de brevet français publié le 5.2.1999 n° 2766708.

L'invention est également relative à un comprimé constitué, au moins en partie, par une composition pharmaceutique flottante telle que définie ci-dessus.

De manière générale, un tel comprimé peut consister en un comprimé à double noyau ou encore un comprimé multicouches.

Selon un premier aspect, un comprimé selon l'invention est caractérisé en ce qu'il s'agit d'un composé bicouche constitué d'une première couche de phase active et d'une seconde couche de phase non active.

Selon un premier mode de réalisation, la première et la seconde couche ont une surface de contact commune.

Selon un second mode de réalisation, la phase active et la phase non active sont séparées par une couche de polymère.

Selon un second aspect, un comprimé selon l'invention est caractérisé en ce qu'il s'agit d'un comprimé tricouche constitué d'une couche de phase active placée entre une première et une seconde couche de phase non active.

La phase active peut être séparée de la première et de la seconde phase non active par une pellicule de polymère (pelliculage).

Selon un troisième aspect, un comprimé selon l'invention consiste en un comprimé tricouche constitué d'une couche de phase non active placée entre une première et une seconde couche de phase active.

Selon un quatrième aspect, un comprimé selon l'invention se présente sous la forme de microgranules.

Selon encore un autre mode de réalisation d'un comprimé selon l'invention, celui-ci est constitué d'un noyau interne de phase active et d'une couche externe de phase non active recouvrant le noyau interne (comprimé double noyau).

Dans un tel mode de réalisation, le noyau interne de phase active peut être séparé de la couche externe de phase non active par un pelliculage, de manière à éviter un contact direct entre la phase active et la phase non active et en conséquence une interaction entre les excipients de la phase non active et le ou les principes actifs contenus dans la phase active.

Selon encore un autre mode de réalisation, la phase active peut elle-même être constituée d'un noyau interne et d'une phase externe:
a) le noyau interne étant constitué d'une composition comprenant au moins un principe actif et, à titre d'agents désintégrants, de l'hydroxypropylméthylcellulose, de l'hydroxypropylcellulose fortement substitué et/ou de l'éthylcellulose dans des quantités inférieures à 15% en poids de la phase active et sous une forme permettant d'obtenir un effet désintégrant tout en évitant la formation d'un réseau continu;
b) la phase externe comprenant au moins un composé à fort effet désintégrant et une quantité efficace du ou des principes actifs compris dans le noyau interne.

La phase externe comprend au moins une quantité efficace d'un ou plusieurs principes actifs et au moins un composé à fort effet désintégrant.

Un tel mode de mise en oeuvre de la présente invention est particulièrement avantageux car il permet de libérer rapidement une forte dose de principe actif par dissolution de la phase externe puis de libérer de manière retardée le ou les principes actifs contenus dans le noyau interne.

Une telle forme pharmaceutique sera avantageusement mise en oeuvre par exemple pour les principes actifs anti-acides décrits précédemment ou tout autre principe actif nécessitant une libération multimodale, par exemple bimodale, qui se caractérise par une libération rapide suivie d'une libération lente du principe actif.

Dans ce mode de réalisation particulier d'un comprimé selon l'invention, la phase non active est ajoutée sur la surface externe du double noyau , par exemple par compression.

Dans encore un autre mode de réalisation particulier d'un comprimé selon l'invention, ce comprimé est adapté à la libération du ou des principes actifs à un niveau post-gastrique, par exemple dans le tractus intestinal et dans le jugénum.

Selon un premier aspect, un tel comprimé comprend, en plus de la phase active et de la phase non-active telle que décrite précédemment, un polymère possédant des propriétés muco-adhésives. De manière tout à fait préférée, le polymère possédant des propriétés muco-adhésives recouvre la surface externe de la couche non-active. Ce mode de réalisation particulier d'un comprimé selon l'invention permet d'obtenir des propriétés d'adhésion du comprimé aux muqueuses tout en maintenant un contrôle précis de la libération du ou des principes actifs contenus dans la phase active.

Selon ce mode de réalisation, un tel comprimé adhère aux muqueuses, préférentiellement les muqueuses post-gastriques, du côté externe de la phase non-active tandis que le principe actif est libéré de la phase active qui est directement en contact avec le milieu aqueux extérieur. Dans le cas d'un composé tricouche constitué d'une couche de phase active placée entre une première et une seconde couche de phase non active, chacune des surfaces externes des deux couches de phase inactive est recouverte d'un polymère possédant des propriétés muco-adhésives.

Selon ce mode de réalisation particulier d'un comprimé selon l'invention, on obtient une libération du ou des principes actifs par érosion et diffusion de la couche active, sans érosion simultanée de la couche non active recouverte d'un polymère qui adhère aux muqueuses.

Lorsque le comprimé se présente sous la forme de micro-granules, chacun des micro-granules est alors recouvert d'un polymère possédant des propriétés muco-adhésives.

Préférentiellement, le polymère possédant des propriétés muco-adhésives est choisi parmi les celluloses et leurs dérivés, le carbomer(carbopol) et ses dérivés, la povidone et ses dérivés ou encore le polyvinylacétate(PVA) et ses dérivés.

Selon un second aspect, un comprimé selon l'invention peut être rendu gastro-résistant, lorsque le ou les principes actifs contenus dans la phase active doivent être libérés en aval de l'estomac, au niveau post-gastrique comme par exemple au niveau de l'intestin ou du jugénum.

Selon cet aspect particulier d'un comprimé selon l'invention, le comprimé est enrobé d'une couche d'un polymère gastro-résistant capable de se solubiliser à un pH supérieur au pH de l'estomac, c'est-à-dire à un pH supérieur à 4, et de manière tout à fait préférée à un pH supérieur à 2.

Lorsque le comprimé se présente sous la forme de microgranules, chacun des microgranules est enrobé d'une couche d'un polymère gastro-résistant.

Préférentiellement, le polymère gastro-résistant est choisi parmi l'acétate phthalate de cellulose, le phthalate d'hydropropylméthylcellulose (encore appelé phthalate d'hypromellose) ou encore un copolymère de l'acide méthacrylique.

Dans un mode de réalisation tout à fait préféré d'un comprimé gastro-résistant selon l'invention, un tel comprimé comprendra, en combinaison, à la fois un polymère possédant des propriétés muco-adhésives et un polymère gastro-résistant, conformément au mode de réalisation décrit ci-dessus.

Un comprimé gastro-résistant selon l'invention est particulièrement adapté à la libération de principes actifs à fenêtre de libération qui sont sensibles à un pH acide et qui peuvent en conséquence être dégradés par le pH acide du suc gastrique.

Un comprimé gastro-résistant selon l'invention est aussi particulièrement adapté lorsque la libération d'un principe actif dont les sites cibles sont localisés au niveau de l'intestin est recherchée.

Selon encore un autre aspect, un comprimé gastro-résistant selon l'invention est particulièrement avantageux dans tous les cas où la libération du ou des principes actifs est recherchée au niveau post-gastrique.

Un comprimé gastro-résistant selon l'invention comprendra de préférence un ou plusieurs principes actifs choisis parmi les principes actifs suivants:
- Acyclovir et ses sels
- Ranitidine et ses sels
- Alfusozine et ses sels
- Metformine et ses sels
- Nicardipine et ses sels
- Antibiotiques et plus particulièrement les β-lactamines et notamment le CEFACLOR et ses sels ou encore Ciprofloxacine,
- Hormones et plus particulièrement les oestrogènes et progestatifs tels que par exemple l'oestradiol et la progestérone;
- Méthyldopa et ses sels
- Levodopa,
- Inosine
- Verapamil
- Nifedipine
- Tribavidine
- Zidovudine
- Ketanserine
- Loperamid
- Cimetidine
- Misoprostol
- Omeprazol

L'invention a encore pour objet un procédé de préparation d'un comprimé tel que défini ci-dessus, caractérisé en ce qu'au moins une couche de phase active et au moins une couche de phase non active sont mises en contact puis solidarisées entre elles par compression.

Selon encore un autre aspect, la phase active peut être constituée de microgranules contenant une association du ou des principes actifs avec les excipients. Le comprimé correspondant est alors constitué d'un noyau de phase active comprenant lesdits microgranules entourée d'une couche externe constituée de la phase non active. Le cas échéant, le noyau interne de phase active et la couche externe de phase non active sont séparés par une pellicule de polymère, selon des techniques bien connues de l'homme du métier.

Un comprimé gastro-résistant selon l'invention est fabriqué préférentiellement par des techniques mettant en oeuvre un lit d'air fluidisé, une turbine de pelliculage ou encore une technique de Mélange-Granulation-Séchage (MGS), bien connues de l'homme du métier.

De manière générale, l'homme du métier peut déterminer, pour chaque principe actif, les quantités adéquates d'excipients, en particulier en utilisant les tests de dissolution décrits dans la « Pharmacopée Européenne » (1997, Méthode de Pharmacotechnie, pages 127 à 130). Ces tests sont réalisés en disposant la forme pharmaceutique contenant le principe actif dans un appareil à palettes, à panier, ou à flux continu, et en observant les cinétiques de libération du principe actif.

L'homme du métier peut ainsi déterminer de manière simple et fiable les rapports principe(s) actif(s)/excipient(s) et moduler la libération (immédiate ou prolongée) en fonction des besoins thérapeutiques.

L'invention est en outre illustrée par les figures et les exemples suivants:

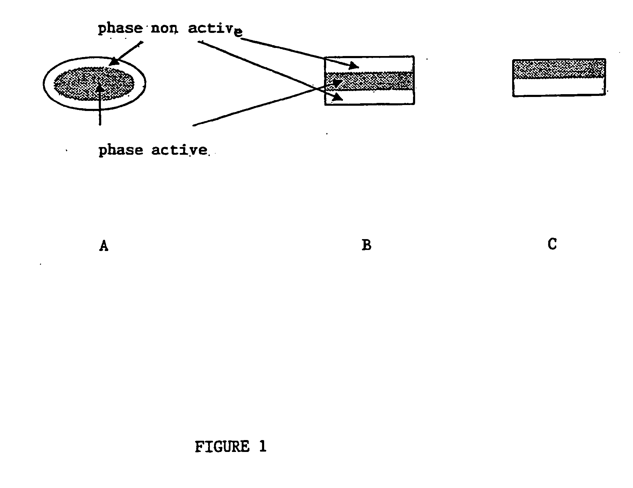

La figure 1 illustre plusieurs modes de réalisation d'un comprimé flottant selon l'invention:
   A : comprimé constitué d'un noyau interne de phase active et d'une couche externe de phase non active.
   B : comprimé tricouche constitué d'une couche de phase active localisée entre deux couches de phase non active.
   C : comprimé bicouche constitué d'une couche de phase active et d'une couche de phase non active.

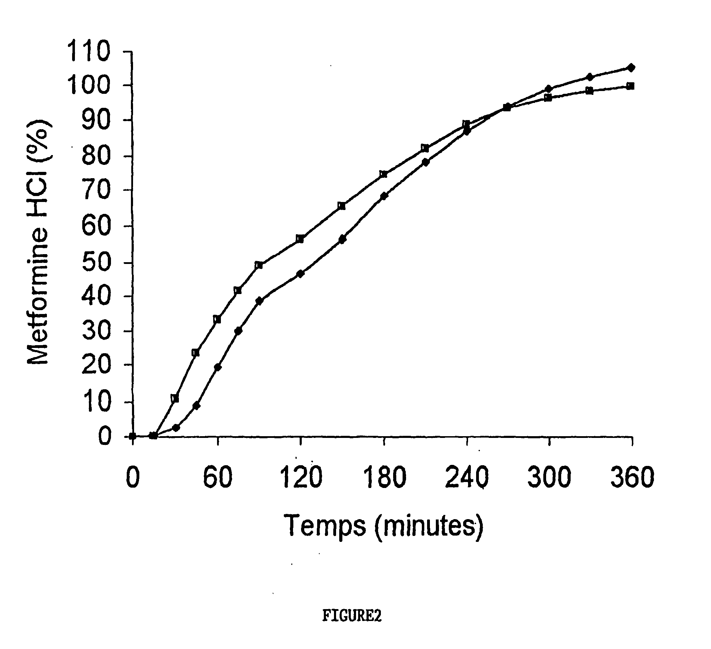

La figure 2 illustre les résultats comparatifs de profil de dissolution d'un comprimé flottant bicouche selon l'invention, et d'un comprimé constitué uniquement d'une couche de phase active.

En abscisse, pourcentage du principe actif Metformine libellé.

En ordonnées, temps écoulé après contact des comprimés avec un milieu aqueux.
- Les carrés représentent les résultats obtenus avec la phase active du comprimé décrit à l'exemple 1.
- Les losanges représentent les résultats obtenus avec le comprimé de l'exemple 1.

### EXEMPLE 1-

### Comprimé à libération programmée contenant du chlorhydrate de metformine.

| Nom des composants | | Quantité (en %) |
|---|---|---|
| Couche active | | |
| | • Metformine HCl | 51,33 |
| | • Carbomère (Carbopol®974) | 3,02 |
| | • Hydroxypropylcellulose fortement substitué | 4,53 |
| | (Klucel® HXF) | |
| | • Stéarate de magnésium | 0,06 |

| Couche non active | | |
|---|---|---|
| | • Hydroxypropylméthylcellulose | 24,64 |
| | (Metolose® 90SH4000SR) | 9,20 |
| | • Citrate monosodique anhydre | 7,23 |
| | • Bicarbonate de sodium (13/50) | |

Composition présentée sous forme de comprimés tricouches ou bicouches.

### Mode de préparation:

a) Couche active: granulation par voie humide.
   - Dans un mélangeur granulateur de type ROTOLAB® ou équivalent, introduire le chlorhydrate de metformine et le CARBOPOL®.
   - Mélanger à l'aide de la tripale pendant 3 minutes.
   - Débuter l'opération de mouillage en incorporant de l'eau purifiée comme solution de mouillage.
   - Granuler l'ensemble jusqu'à obtention d'un grain de qualité satisfaisante.
   - Sécher le grain
   - Calibrer le grain sur granulateur oscillant ERWEKA AR 401 ou équivalent.
   - Mélanger au grain calibré l'hydroxypropylcellulose puis le stéarate de magnésium dans un mélangeur adapté.
b) Couche non active.
   - Un mélange homogène est réalisé entre les différents composants de cette couche.

Les deux compositions a) et b) ainsi préparées servent à alimenter la machine à comprimer équipée de poinçons de format 13,5R17.

Les comprimés issus de cette fabrication sont soit des bicouches ou soit des tricouches de masse moyenne 974 mg par unité.

### EXEMPLE 2:

### Comprimé à libération programmée contenant du flutamide.

| Nom des composants | | Quantité (en %) |
|---|---|---|
| Couche active | | |
| | • Flutamide | 70,00 |
| | • Povidone K90F | 2,00 |
| | • Hydroxypropylméthylcellulose | 1,00 |
| | (Metolose® 90SH4000SR) | 6,25 |
| | • Stéarate de magnésium | 0,25 |

| Couche non active | | |
|---|---|---|
| | • Hydroxypropylméthylcellulose | 20 |
| | (Metolose® 90SH100000SR) | |
| | • Bicarbonate de potassium | 6,75 |

Dans ce cas, la couche active est fabriquée par un procédé de granulation humide et comprimée sur un poinçon de diamètre 12 mm.

La couche non active sert à alimenter une machine à comprimer en vu de la fabrication de comprimés double noyau.

### EXEMPLE 3:

### Etude du profil de dissolution d'une composition pharmaceutique selon l'invention comparée au profil de dissolution de composition pharmaceutique conventionnelle.

Les profils de dissolution de deux compositions pharmaceutiques ont été étudiés, respectivement pour :
- un comprimé d'une composition pharmaceutique selon l'invention, conforme à l'exemple 1:

- un comprimé contenant uniquement la phase active d'une composition pharmaceutique conforme à l'exemple 1.

Les comprimés de chacune des compositions pharmaceutiques ci-dessus ont été immergés dans un milieu tampon ajusté à pH 6,8 dans un dispositif d'analyse de dissolution classique à pales, la vitesse des pales étant fixée à 100 tours/minute et le volume du milieu étant de 1000 ml, à une température de 37°C.

Les comprimés testés sont des comprimés obtenus selon un format de compression de 13,5 R17 . Lors du test, les comprimés sont « lestés », c'est-à-dire placés dans un panier de type JAGO, lui-même déposé au fond de la cuve de dissolution.

Les résultats sont reportés à la figure 2.

Comme on peut le voir, les profils de dissolution du principe actif chlorhydrate de metformine sont identiques pour les deux comprimés testés.

Ces résultats montrent que l'adjonction de la phase non active constitutive d'un comprimé selon l'invention est sans effet observable sur le profil de dissolution du principe contenu dans la phase active.

### EXEMPLE 4:

### Etude de la durée et de flottaison d'un comprimé constitué d'une composition pharmaceutique selon l'invention.

Le comprimé testé est un comprimé tricouche tel que décrit à la figure 1B, préparé essentiellement comme décrit à l'exemple 1.

La couche de phase active est comprimée entre deux couches de phase non active de même composition.

La phase non active comprend un rapport citrate monosodique/bicarbonate de sodium égal à 65/35. La résistance à la rupture des comprimés est d'environ 390 Newtons.

Les comprimés ont été immergés dans respectivement :
- 100 ml d'eau. R conforme à la section « Réactifs » de la Pharmacopée, à la température de 37°C
- 100 ml d'HCl à 0,1N à 37°C.

Dans l'eau R à 37°C, le temps de latence avant flottaison des comprimés est de 47 ». Ce temps de latence est de 29 secondes dans 100 ml d'HCl à 0,1N.

Dans les deux milieux aqueux, les comprimés flottent après plus de 16 heures.

## Revendications

1. Composition pharmaceutique flottante **caractérisée en ce qu'**elle est constituée d'au moins :
a) une première phase, dite active, comprenant au moins un principe actif et, à titre d'agents désintégrants, de l'hydroxypropylméthyl cellulose, de l'hydroxypropylcellulose fortement substituée et/ou de l'éthylcellulose dans des quantités inférieures à 15% en poids de la phase active et sous une forme permettant d'obtenir un effet désintégrant tout en évitant la formation d'un réseau continu ;
b) une seconde phase, dite non active, comprenant au moins un système générateur d'un gaz et au moins un polymère hydrophile,
la phase active comprenant une quantité de principe(s) actif(s) d'au moins 80%, préférentiellement d'au moins 85%, et encore plus préférentiellement d'au moins environ 90% en poids de la phase active.

2. Composition selon la revendication 1, **caractérisée en ce que** le polymère hydrophile de la phase non active est choisi parmi les substances polysaccharidiques et les substances apparentées, et les substances protéiques.

3. Composition selon la revendication 2, **caractérisée en ce que** les substances polysaccharidiques et les substances apparentées sont choisies parmi les galactomannanes et ses dérivés, l'amidon et ses dérivés, la gomme arabique, la gomme adragante, les pectines et ses dérivés, les alginates, la cellulose et ses dérivés, les dextrans de haut poids moléculaire, ou encore le xanthane et ses dérivés.

4. Composition selon la revendication 2, **caractérisée en ce que** les substances protéiques sont choisies parmi la gélatine et ses produits dérivés purifiés.

5. Composition selon la revendication 1, **caractérisée en ce que** le polymère hydrophile de la phase non active est choisi parmi les poloxamères, les polyéthylène glycols de haut poids moléculaire ou encore les polymères des acides acryliques ou méthacryliques et leurs dérivés.

6. Composition selon l'une des revendications 1 à 5, **caractérisée en ce que** le système générateur de gaz de la phase inactive est un système générateur de dioxyde de carbone.

7. Composition selon la revendication 6, **caractérisée en ce qu'**il s'agit d'un système générateur de dioxyde de carbone comprenant un carbonate de métal alcalin ou alcalino-terreux, tel que le carbonate de calcium ou encore un bicarbonate de métal alcalin, tel que le bicarbonate de sodium ou le bicarbonate de potassium.

8. Composition selon la revendication 7, **caractérisée en ce que** ledit carbonate de métal alcalin ou alcalino-terreux ou ledit bicarbonate est en association avec un acide choisi parmi les acides mono- et polycarboxyliques ou un sel partiel d'un acide polycarboxylique.

9. Composition selon l'une des revendications 1 à 8, **caractérisée en ce que** la phase non active comprend de 10 à 70% en poids de ladite phase d'un polymère hydrophile.

10. Composition selon l'une des revendication 1 à 9, **caractérisée en ce que** la phase non active comprend de 5 à 60% en poids de ladite phase d'un système générateur de gaz.

11. Composition selon l'une des revendications 1 à 10, **caractérisée en ce que** la phase active comprend au moins un principe actif choisi parmi les principes actifs se présentant sous une forme ionique en milieu acide, et les principes actifs qui sont dégradés par un pH acide.

12. Comprimé constitué, au moins en partie, par une composition selon l'une des revendications 1 à 11.

13. Comprimé selon la revendication 12, **caractérisé en ce qu'**il s'agit d'un composé bicouche constitué d'une première couche de phase active et d'une seconde couche de phase non active.

14. Comprimé selon la revendication 13, **caractérisé en ce qu'**il s'agit d'un comprimé tricouche constitué d'une couche de phase active placée entre une première et une seconde couche de phase non active.

15. Comprimé selon la revendication 12, **caractérisé en ce qu'**il s'agit d'un comprimé tricouche constitué d'une couche de phase non active placée entre une première et une seconde couche de phase active.

16. Comprimé selon la revendication 12, **caractérisé en qu'**il est constitué d'un noyau interne de phase active et d'une couche externe de phase non active recouvrant le noyau interne.

17. Comprimé selon la revendication 12, **caractérisé en ce qu'**il se présente sous la forme de micro-granules.

18. Comprimé selon la revendication 12, **caractérisé en ce que** la phase active est un double noyau constituée d'un noyau interne et d'une phase externe,
a) le noyau interne étant constitué d'une composition comprenant au moins un principe actif et, à titre d'agents désintégrants, de l'hydroxypropylméthyl cellulose, de l'hydroxypropylcellulose fortement substituée et/ou de l'éthylcellulose dans des quantités inférieures à 15 pour cent en poids de la phase active et sous une forme permettant d'obtenir un effet désintégrant tout en évitant la formation d'un réseau continu ;
b) la phase externe comprenant au moins un composé à fort effet désintégrant et une quantité efficace du ou des principes actifs compris dans le noyau interne.

19. Comprimé selon l'une des revendications 12 à 16, **caractérisé en ce que** la surface externe de la couche non active est recouverte d'un polymère possédant des propriétés muco-adhésives.

20. Comprimé selon la revendication 17, **caractérisé en ce que** chacun des microgranules est recouvert d'un polymère possédant des propriétés muco-adhésives.

21. Comprimé selon l'une des revendications 19 à 20, **caractérisé en ce que** le polymère possédant des propriétés mucoadhésives est choisi parmi :
- les celluloses et leurs dérivés ;
- le carbomer dénommé carbopol et ses dérivés
- la povidone et ses dérivés ;
- le polyvinylacétate dénommé PVA et ses dérivés.

22. Comprimé selon l'une des revendications 12 à 16 et 19, **caractérisé en ce qu'**il est enrobé d'une couche d'un polymère gastrorésistant.

23. Comprimé selon l'une des revendications 17 et 20, **caractérisé en ce que** chaque microgranule est enrobé d'une couche d'un polymère gastrorésistant.

24. Composé selon l'une des revendications 22 et 23, **caractérisé en ce que** le polymère gastro-résistant est choisi parmi l'acétatephtalate de cellulose, le phtalate d'hydroxypropylméthylcellulose dénommé hypromellosephtalate et un copolymère d'acide méthacrylique.

25. Procédé de préparation d'un comprimé selon l'une des revendications 12 à 15, **caractérisé en ce qu'**au moins une couche de phase active et au moins une couche de phase non active sont mises en contact puis solidarisées entre elles par compression.

## Claims

1. Floating pharmaceutical composition **characterised in that** it is constituted by at least :
a) a first phase, termed active phase, comprising at least one active ingredient and, as disintegrating agents, hydroxypropylmethylcellulose, highly substituted hydroxypropylcellulose and/or ethylcellulose, in quantities less than 15% by weight of the active phase and in a form making it possible to obtain a disintegrating effect while preventing the formation of a continuous network,
b) a second phase, termed inactive phase, comprising at least one gas-generating system and at least one hydrophilic polymer,
the active phase comprising a quantity of active ingredient(s) constituting at least 80%, preferably at least 85% and more preferably at least 90% by weight of the active phase.

2. Floating pharmaceutical composition according to claim 1 wherein the hydrophilic polymer of the inactive phase is selected from polysaccharide substances and related substances and protein substances.

3. Composition according to Claim 2, **characterised in that** the polysaccharide substances and related substances are selected from the galactomannans and their derivatives, starch and its derivatives, gum arabic, tragacanth gum, the pectins and their derivatives, the alginates, cellulose and its derivatives, the high molecular weight dextrans or also xanthan and its derivatives.

4. Composition according to Claim 2, **characterised in that** the protein substances are selected from gelatine and its purified derivatives.

5. Composition according to Claim 1, **characterised in that** the hydrophilic polymer of the inactive phase is selected from the poloxamers, the high molecular weight polyethylene glycols or also the polymers of the methacrylic or acrylic acids and their derivatives.

6. Composition according to any one of Claims 1 to 5, **characterised in that** the gals-generating system of the inactive phase is a carbon dioxide generating system.

7. Composition according to Claim 6, **characterised in that** it is a carbon dioxide generating system comprising an alkali or alkaline earth metal carbonate such as calcium carbonate, or also an alkali metal bicarbonate such as sodium bicarbonate or potassium bicarbonate.

8. Composition according to Claim 7, **characterised in that** said alkali or alkaline earth metal carbonate or said bicarbonate is in combination with an acid selected from the mono- and polycarboxylic acids or a partial salt of a polycarboxylic acid.

9. Composition according to any one of Claims 1 to 8, **characterised in that** the inactive phase comprises from 10 to 70% of a hydrophilic polymer by weight of the said phase.

10. Composition according to any one of Claims 1 to 9, **characterised in that** the inactive phase comprises from 5 to 60% of a gas-generating system by weight of the said phase.

11. Composition according to any one of Claims 1 to 10, **characterised in that** the active phase comprises at least one active ingredient selected from the active ingredients present in an ionic form in an acidic medium and the active ingredients which are degraded by an acidic pH.

12. Tablet constituted at least in part by a composition according to any one of Claims 1 to 11.

13. Tablet according to Claim 12, **characterised in that** it is a bilayer composite constituted of a first layer of active phase and a second layer of inactive phase.

14. Tablet according to Claim 13, **characterised in that** it is a triple layer tablet constituted of a layer of active phase placed between a first and second layer of inactive phase.

15. Tablet according to Claim 12, **characterised in that** it is a triple layer tablet constituted of a layer of inactive phase placed between a first and a second layer of active phase.

16. Tablet according to Claim 12, **characterised in that** it is constituted of an internal core of active phase and an external layer of inactive phase coating the internal core.

17. Tablet according to Claim 12, **characterised in that** it is available in the form of micro-granules.

18. Tablet according to Claim 12, **characterised in that** the active phase is a double core constituted of an internal core and an external phase,
a) the internal core being constituted of a composition comprising at least one active ingredient and, as disintegrating agents, hydroxypropylmethylcellulose, highly substituted hydroxypropylcellulose and ethylcellulose in quantities less than 15 percent by weight of the active phase and in a form making it possible to obtain a disintegrating effect while preventing the formation of a continuous network ;
b) the external phase comprising at least one compound with a high disintegrating effect and an efficacious quantity of the active ingredient(s) comprised in the internal core.

19. Tablet according to any one of Claims 12 to 16, **characterised in that** the external surface of the inactive layer is coated with a polymer possessing muco-adhesive properties.

20. Tablet according to Claim 17, **characterised in that** each of the micro-granules is coated with a polymer possessing muco-adhesive properties.

21. Tablet according to to any one of Claims 19 to 20, **characterised in that** the polymer possessing muco-adhesive properties is selected from :
- the celluloses and their derivatives ;
- the carbomer termed carbopol and its derivatives ;
- povidone and its derivatives ;
- polyvinylacetate termed PVA and its derivatives.

22. Tablet according to any one of Claims 12 to 16 and 19, **characterised in that** it is coated with a layer of a gastro-resistant polymer.

23. Tablet according to any one of Claims 17 and 20, **characterised in that** each micro-granule is coated with a layer of a gastro-resistant polymer.

24. Tablet according to any one of Claims 22 and 23, **characterised in that** the gastro-resistant polymer is selected from cellulose acetate phthalate, hydroxypropylmethylcellulose phthalate termed hypromellose phthalate and a methacrylic acid copolymer.

25. Process for the preparation of a tablet according to any one of Claims 12 to 15, **characterized in that** at least one layer of active phase and at least one layer of inactive phase are placed in contact, then cemented by compression.

## Patentansprüche

1. Schwimmende pharmazeutische Zusammensetzung, **dadurch gekennzeichnet, dass** sie sich aus wenigstens
a) einer ersten, aktiv bezeichneten Phase, die wenigstens ein aktives Prinzip und als Zerfallhilfsmittel Hydroxypropylmethylcellulose, hochsubstituierte Hydroxypropylcellulose und/oder Ethylcellulose in Mengen unter 15 Gew.-% der aktiven Phase und in einer Form umfasst, die das Erhalten einer Zerfallswirkung gestattet, bei der die Bildung eines durchgehenden Netzwerks vermieden wird, und
b) einer zweiten, nicht-aktiv bezeichneten Phase zusammensetzt, die wenigstens ein Gaserzeugungssystem und wenigstens ein hydrophiles Polymer umfasst,
wobei die aktive Phase eine Menge an aktivem (aktiven) Prinzip(ien) von mindestens 80 Gew.-%, vorzugsweise mindestens 85 Gew.-% und noch bevorzugter mindestens etwa 90 Gew.-% der aktiven Phase umfasst.

2. Zusammensetzung gemäß Anspruch 1, **dadurch gekennzeichnet, dass** das hydrophile Polymer der nicht-aktiven Phase aus Polysaccharidmaterialien und verwandten Materialien und Proteinmaterialien ausgewählt ist.

3. Zusammensetzung gemäß Anspruch 2, **dadurch gekennzeichnet, dass** die Polysaccharidmaterialien und die verwandten Materialien aus Galactomannanen und ihren Derivaten, Amidon und seinen Derivaten, Gummiarabicum, Traganthgummi, Pektinen und ihren Derivaten, Alginaten, Cellulose und ihren Derivaten, Dextranen mit hohem Molekulargewicht oder auch Xanthan und seinen Derivaten ausgewählt sind.

4. Zusammensetzung gemäß Anspruch 2, **dadurch gekennzeichnet, dass** die Proteinmaterialien aus Gelatine und den von ihr abgeleiteten, gereinigten Produkten ausgewählt sind.

5. Zusammensetzung gemäß Anspruch 1, **dadurch gekennzeichnet, dass** das hydrophile Polymer der nicht-aktiven Phase aus Poloxameren, Polyethylenglykolen mit hohem Molekulargewicht oder auch Acryl- oder Methacrylsäurepolymeren und ihren Derivaten ausgewählt ist.

6. Zusammensetzung gemäß einem der Ansprüche 1 bis 5, **dadurch gekennzeichnet, dass** das Gaserzeugungssystem der inaktiven Phase ein Kohlendioxiderzeugungssystem ist.

7. Zusammensetzung gemäß Anspruch 6, **dadurch gekennzeichnet, dass** es sich um ein Kohlendioxiderzeugungssystem handelt, das ein Alkalimetall- oder Erdalkalimetallcarbonat wie etwa Calciumcarbonat oder auch ein Alkalimetallbicarbonat wie etwa Natriumbicarbonat oder Kaliumbicarbonat umfasst.

8. Zusammensetzung gemäß Anspruch 7, **dadurch gekennzeichnet, dass** das Alkalimetall- oder Erdalkalimetallcarbonat oder -bicarbonat mit einer Säure kombiniert ist, die aus Mono- und Polycarbonsäuren oder einem teilweisen Salz einer Polycarbonsäure ausgewählt ist.

9. Zusammensetzung gemäß einem der Ansprüche 1 bis 8, **dadurch gekennzeichnet, dass** die nicht-aktive Phase 10 bis 70 Gew.-% der Phase an einem hydrophilen Polymer umfasst.

10. Zusammensetzung gemäß einem der Ansprüche 1 bis 9, **dadurch gekennzeichnet, dass** die nicht-aktive Phase 5 bis 60 Gew.-% der Phase an einem Gaserzeugungssystem umfasst.

11. Zusammensetzung gemäß einem der Ansprüche 1 bis 10, **dadurch gekennzeichnet, dass** die aktive Phase wenigstens ein aktives Prinzip umfasst, das aus den aktiven Prinzipien, die in saurem Medium eine ionische Form aufweisen, und den aktiven Prinzipien ausgewählt ist, die durch einen sauren pH abgebaut werden.

12. Tablette, die sich wenigstens zum Teil aus einer Zusammensetzung gemäß einem der Ansprüche 1 bis 11 zusammensetzt.

13. Tablette gemäß Anspruch 12, **dadurch gekennzeichnet, dass** es sich um eine Zweischichtenverbindung handelt, die sich aus einer ersten Schicht aus aktiver Phase und einer zweiten Schicht aus nicht-aktiver Phase zusammensetzt.

14. Tablette gemäß Anspruch 13, **dadurch gekennzeichnet, dass** es sich um eine Dreischichtenzusammensetzung handelt, die sich aus einer Schicht aus aktiver Phase zwischen einer ersten und einer zweiten Schicht aus nicht-aktiver Phase zusammensetzt.

15. Tablette gemäß Anspruch 12, **dadurch gekennzeichnet, dass** es sich um eine Dreischichtenzusammensetzung handelt, die sich aus einer Schicht aus nicht-aktiver Phase zwischen einer ersten und einer zweiten Schicht aus aktiver Phase zusammensetzt.

16. Tablette gemäß Anspruch 12, **dadurch gekennzeichnet, dass** sie sich aus einem inneren Kern aus aktiver Phase und einer den inneren Kern überziehenden äußeren Schicht aus nicht-aktiver Phase zusammensetzt.

17. Tablette gemäß Anspruch 12, **dadurch gekennzeichnet, dass** sie die Form eines Mikrogranulats aufweist.

18. Tablette gemäß Anspruch 12, **dadurch gekennzeichnet, dass** die aktive Phase ein Doppelkern ist, der sich aus einem inneren Kern und einer äußeren Phase zusammensetzt, wobei
a) der innere Kern sich aus einer Zusammensetzung zusammensetzt, die wenigstens ein aktives Prinzip und als Zerfallhilfsmittel Hydroxypropylmethylcellulose, hochsubstituierte Hydroxypropylcellulose und/oder Ethylcellulose in Mengen unter 15 Gew.-% der aktiven Phase und in einer das Erhalten einer Zerfallhilfswirkung unter Vermeiden der Bildung eines durchgehenden Netzwerks gestattenden Form umfasst,
b) die äußere Phase wenigstens eine Verbindung mit starker Zerfallhiffswirkung und eine wirksame Menge des im inneren Kern enthaltenen aktiven Prinzips oder der aktiven Prinzipen umfasst.

19. Tablette gemäß einem der Ansprüche 12 bis 16, **dadurch gekennzeichnet, dass** die äußere Oberfläche der nicht-aktiven Schicht mit einem mucoadhäsive Eigenschaften besitzenden Polymer überzogen ist.

20. Tablette gemäß Anspruch 17, **dadurch gekennzeichnet, dass** jede Mikrogranalie mit einem mucoadhäsive Eigenschaften besitzenden Polymer überzogen ist.

21. Tablette gemäß einem der Ansprüche 19 bis 20, **dadurch gekennzeichnet, dass** das mucoadhäsive Eigenschaften besitzende Polymer aus
- Cellulosen und ihren Derivaten;
- Carbomer, Carbopol gennant, und seinen Derivaten;
- Povidon und seinen Derivaten und
- Polyvinylacetat (PVA genannt) und seinen Derivaten
ausgewählt ist.

22. Tablette gemäß einem der Ansprüche 12 bis 16 und 19, **dadurch gekennzeichnet, dass** sie mit einer Schicht aus einem magensaftresistenten Polymer umhüllt ist.

23. Tablette gemäß einem der Ansprüche 17 und 20, **dadurch gekennzeichnet, dass** jede Mikrogranalie mit einer Schicht aus einem magensaftresistenten Polymer umhüllt ist.

24. Tablette gemäß einem der Ansprüche 22 und 23, **dadurch gekennzeichnet, dass** das magensaftresistente Polymer aus Celluloseacetatphthalat, Hydroxypropylmethylcellulosephthalat, Hypromellosephthalat genannt, und einem Methacrylsäurecopolymer ausgewählt ist.

25. Verfahren zur Herstellung einer Tablette gemäß einem der Ansprüche 12 bis 15, **dadurch gekennzeichnet, dass** wenigstens eine Schicht aus aktiver Phase und wenigstens eine Schicht aus nicht-aktiver Phase in Kontakt gebracht und dann durch Verpressen miteinander verfestigt werden.
